Europäisches Patentamt

European Patent Office

Office européen des brevets

(19)

(11) Numéro de publication : **0 235 138**
**B1**

(12) **FASCICULE DE BREVET EUROPÉEN**

(45) Date de publication du fascicule du brevet :
**02.11.89**

(51) Int. Cl.⁴ : **A 61 B 17/60**

(21) Numéro de dépôt : **85905660.8**

(22) Date de dépôt : **07.11.85**

(86) Numéro de dépôt international :
**PCT/IT 85/00045**

(87) Numéro de publication internationale :
**WO/8602822 (22.05.86 Gazette 86/11)**

(54) **APPAREILLAGE POUR LA STABILISATION DES FRACTURES OSSEUSES.**

(30) Priorité : **08.11.84 IT 8566084**

(43) Date de publication de la demande :
**09.09.87 Bulletin 87/37**

(45) Mention de la délivrance du brevet :
**02.11.89 Bulletin 89/44**

(84) Etats contractants désignés :
**AT BE CH DE FR GB IT LI LU NL SE**

(56) Documents cités :
**EP-A- 0 140 591**
**CH-A- 638 390**
**DE-A- 3 118 397**
**FR-A- 2 338 692**
**FR-A- 2 439 002**
**FR-A- 2 517 535**
**US-A- 2 393 831**
**US-A- 2 477 562**

(73) Titulaire : **CASTAMAN, Enrico**
**Contra S. Bortolo, 59**
**I-36100 Vicenza (IT)**

**BORGHETTINI, Lino**
**Via Battaglione Aosta, 14**
**I-36100 Vicenza (IT)**

(72) Inventeur : **CASTAMAN, Enrico**
**Contra S. Bortolo, 59**
**I-36100 Vicenza (IT)**
Inventeur : **BORGHETTINI, Lino**
**Via Battaglione Aosta, 14**
**I-36100 Vicenza (IT)**

(74) Mandataire : **Bettello, Luigi, Dott. Ing.**
**Via Col d'Echele, 25**
**I-36100 Vicenza (IT)**

Jouve, 18, rue St-Denis, 75001 Paris, France

EP 0 235 138 B1

## Description

La présente invention a pour objet un appareillage pour la stabilisation des fractures osseuses.

On sait que depuis longtemps le problème de la stabilisation des fractures osseuses à l'aide de moyens mécaniques destinés à remplacer les plâtres devenus obsolètes par suite de leur encombrement et de leur caractère peu fonctionnel, a été étudié par différentes équipes chirurgicales dans les principaux pays du monde.

Jusqu'en 1942 Otto STADER aux ETATS-UNIS a proposé (cf. US-A 2 393 831) de fixer les fractures des os longs à l'aide d'un dispositif pourvu de clous transosseux ; toutefois un tel dispositif présente un poids considérable et se révèle peu souple à l'usage du fait qu'il ne permet pas de réduire la fracture suivant des plans différents, frontaux et sagittal, pour amener en alignement correct les deux tronçons osseux à souder. De ce fait résultent la nécessité d'interventions chirurgicales gênantes pour le patient, ainsi que l'exposition prolongée à un appareillage radiologique en vue de la vérification de l'alignement des deux tronçons.

En 1945 Roger ANDERSON aux Etat-Unis a proposé (cf. US-A 2 477 562) une table opératoire à laquelle est associé un dispositif à tiges traversantes pour la réduction des fractures ; cependant cet ensemble implique pour le patient une longue période d'immobilisation avec tous les inconvénients qui en découlent.

En 1976 un groupe de chercheurs des Instituts français INSERM et CERCA ont proposé (cf. FR-A 2 338 692) un appareil équipé d'une part de mâchoires mobiles le long de deux guides parallèles articulés, d'autre part de mâchoires à ressort, immobilisées par friction. On notera cependant qu'un tel appareil se révèle encombrant et complexe, en nécessitant des recherches compliquées et une série de manœuvres pour permettre la réduction simultanée de la fracture dans les deux plans vertical et horizontal.

En 1978 l'Américain Richard Frederick KRONNER a proposé (cf. FR-2 439 002) un appareillage pour la réduction et l'immobilisation des fractures qui s'est toutefois révélé très lourd, en présentant un encombrement important et un montage malaisé.

En 1979 un groupe de chercheurs tchèques a proposé (cf. CH-A 738 390) un dispositif pour la réduction des fractures muni d'éléments montés à coulissement le long de guides parallèles. Les mâchoires de blocage sont d'un type très simple et ne permettent pas les mouvements en inclinaison et en longueur, ce qui se répercute sur la facilité de réduction des fractures.

En 1981 Rudolf KLEINING a proposé (cf. DE-A 3 118 397) un dispositif drastiquement simplifié qui ne comporte absolument aucune articulation de réglage externe, si bien qu'il est impossible de réduire une fracture après avoir mis en œuvre l'appareillage. Celui-ci n'est ainsi susceptible d'être utilisé que pour des fractures déjà réduites qui ne nécessitent aucune autre intervention une fois l'appareillage mis en place.

Toujours en 1981 Juan Lazo ZBIKOWSKI a proposé (cf. FR-A 2 517 535) un dispositif pourvu de vis traversantes alignées qui sont maintenues par des mâchoires qui présentent un encombrement et un poids considérables, qui ne comportent pas une grande stabilité de retenue à distance et qui ne permettent pas de réduire simultanément les fractures sur deux plans, ce qui constitue un grave inconvénient pour le patient et pour le chirurgien, l'un et l'autre soumis pendant un temps prolongé à des expositions radiologiques destinées à vérifier l'alignement des tronçons osseux.

Enfin en 1981 les présents Demandeurs, MM. CASTAMAN et BORGHETTINI, ont proposé (cf. IT-A 85609/81) un dispositif pour la stabilisation des fractures des os longs, qui offre une grande possibilité de réduction desdites fractures suivant des plans différents, une stabilité mécanique optimale et une grande souplesse d'emploi, en même qu'un poids et un encombrement réduits, tous ces points constituant des avantages notables pour le patient comme pour le chirurgien.

La présente invention a pour objet des perfectionnements apportés au dispositif italien qui précède, en ce qui concerne aussi bien le mode de fixation des tiges transosseuses sur la structure des barres porteuses que les éléments constitutifs de chacune de ces barres, et ce en vue d'améliorer la stabilité de l'appareillage en cours d'utilisation et la possibilité de réglage des pièces constitutives de celui-ci lors de son application sur le membre du patient.

L'un des inconvénients rencontrés en pratique réside dans la difficulté d'assurer une retenue sûre dans le temps de la position des différentes tiges métalliques, éventuellement filetées, qui sont destinées à être insérées dans les fragments osseux élémentaires en vue de maintenir ceux-ci en position jusqu'à ce que le soudage soit intervenu. Un autre inconvénient auquel on se heurte parfois dans l'utilisation de l'appareillage de blocage sus-mentionné d'origine italienne est dû au fait qu'à l'endroit de la liaison entre la partie de guidage et la partie filetée, la barre qui porte les tiges transosseuse risque de se rompre avec des conséquences très graves sur la guérison du patient.

Tous ces inconvénients sont éliminés moyennant l'adoption de l'appareillage perfectionné suivant l'invention, du fait que le mécanisme de blocage des éléments de serrage qui fixe les tiges traversantes transosseuses aux barres porteuses est réalisé au moyen d'un étrier accessoire et d'une rondelle tous deux revêtus d'une garniture déformable, tandis que la partie de guidage de la barre porteuse est en outre prévue à surface rugueuse afin de garantir une fixation pratiquement inamovible des tiges.

Le raccordement curviligne entre la partie de guidage et la partie filetée de la barre porteuse élimine de manière complète le risque de rupture consécutif aux sollicitations qu'on rencontre usuellement une fois que l'appareillage est employé, en garantissant ainsi une sécurité maximale tout au long de la durée d'usage de cet appareillage.

L'invention vise également des perfectionnements concernant la forme ovoïdale de la partie filetée de la barre porteuse, cette forme permettant d'orienter, dans le sens torsionnel de la manière la mieux appropriée, les deux parties qui constituent ladite barre, en fonction des caractéristiques particulières du patient.

Une autre disposition remarquable est constituées par le fait que l'une seulement des deux barres porteuses est fixée à l'articulation intermédiaire à l'aide d'un système télescopique réglable, tandis que l'autre barre constitue une pièce unique avec l'étrier de l'articulation précitée. Cette disposition simplifie de manière sensible les manœuvres destinées à l'adaptation de l'appareillage au cas spécifique du patient sur lequel il est appliqué, en même temps qu'elle augmente la stabilité globale de celui-ci.

Suivant une autre forme de réalisation on prévoit que l'appareillage est réalisé avec une série de tiges disposées suivant un plan perpendiculaire à une seconde série, ce qui permet l'emploi du dispositif pour des fractures épiphysaires du fait qu'il permet de disposer une première série de tiges à travers l'épiphyse osseuse suivant un plan pratiquement perpendiculaire à celui de la série de tiges qui pénètrent à travers la diaphyse osseuse.

Le dessin annexé, donné à titre d'exemple, permettra de mieux comprendre l'invention, les caractéristiques qu'elle présente et les avantages qu'elle est susceptible de procurer :

Fig. 1 est une vue en élévation d'un appareillage suivant l'invention, pourvu de deux séries de tiges alignées.

Fig. 2 est la vue de côté correspondante.

Fig. 3 est une vue de détail en élévation montrant la zone de liaison entre la partie filetée et la partie de guidage de l'une des barres porteuses.

Fig. 4 est une coupe transversale suivant le plan indiqué en IV-IV en fig. 3.

Fig. 5 est une vue de côté montrant, préalablement à leur assemblage, les éléments constitutifs de l'un des mécanismes à vis qui assure le blocage des tiges.

Fig. 6 est une vue en élévation d'une forme de réalisation de l'appareillage suivant l'invention, destinée aux fractures épiphysaires.

Fig. 7 en est la vue de côté.

Fig. 8 montre de manière très schématique une jointure osseuses avec l'indication des points pour la mise en place des tiges de l'appareillage suivant fig. 6 et 7.

Fig. 9 est la vue de côté correspondante.

Fig. 10 est une vue de côté représentant schématiquement une structure osseuse avec indication des points pour le passage des tiges dans le cas d'une fracture intermédiaire.

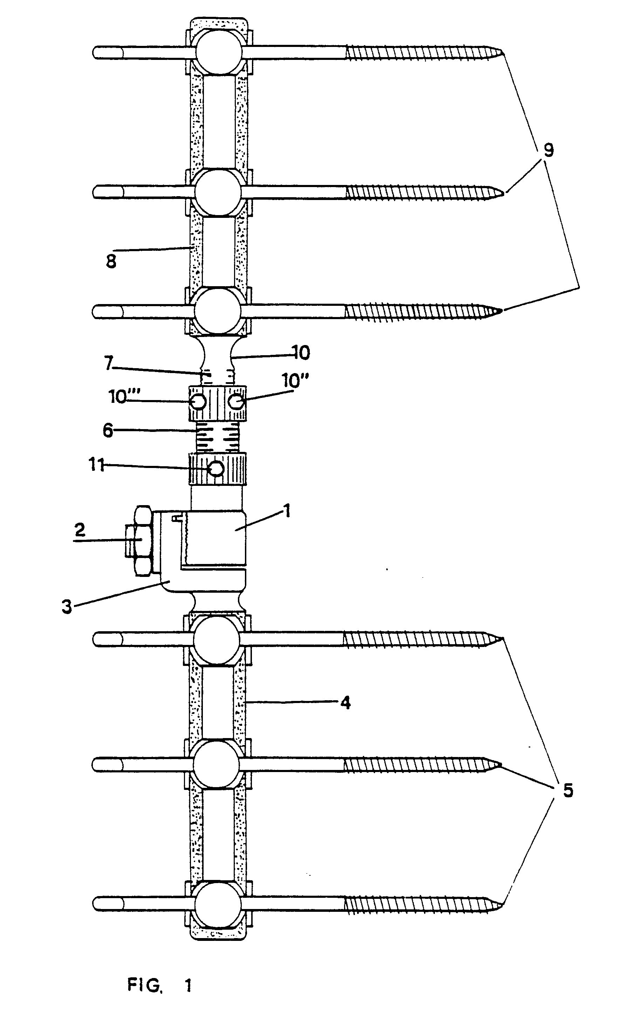

Le dispositif ou appareillage illustré en fig. 1 à 4 est particulièrement adapté à la stabilisation des fractures d'os longs.

Il comprend un dé d'articulation 1 qui est relié par une vis 2 à un secteur 3, lequel est solidaire d'une barre de guidage 4 destinée à porter les tiges 5. A l'opposé de la barre 4 le dé 1 porte une douille filetée 6 sur laquelle vient se fixer par vissage un axe fileté 7 qui est solidaire d'une seconde barre de guidage 8, laquelle est destinée à porter une série de tiges 9.

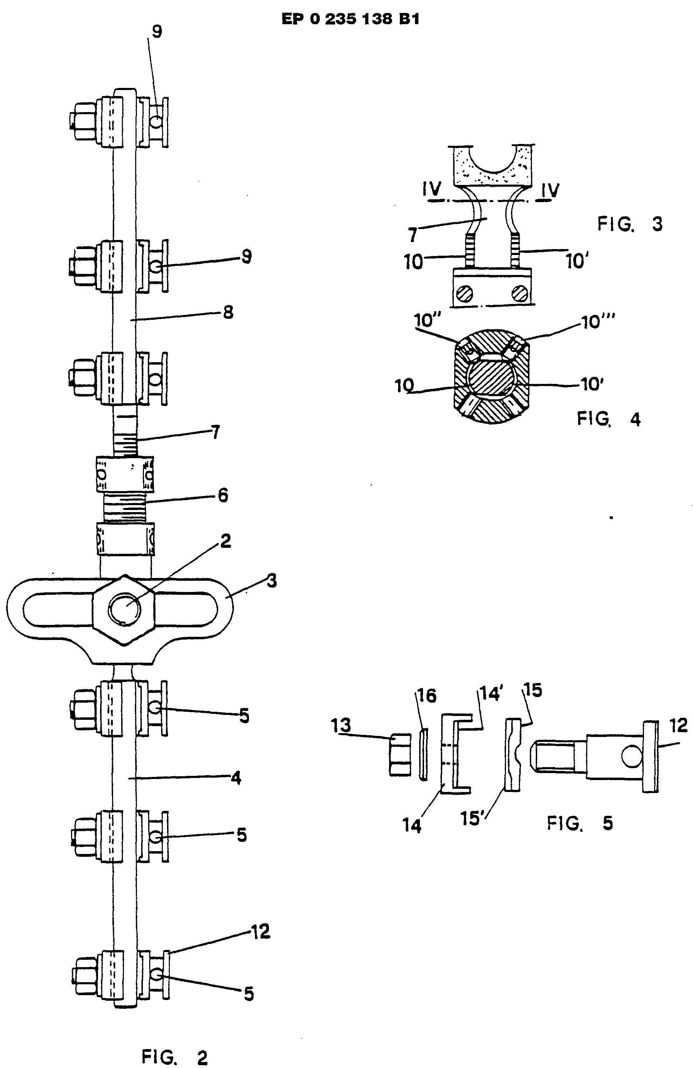

On observera (fig. 3 et 4) que l'axe fileté 7 présente une section ovoïdale au niveau de laquelle le filetage est limité à deux secteurs opposés 10 et 10'. Sur l'une des parois courbes non filetées viennent s'engager les vis-pointeau sans tête 10'' et 10''' qui assurent le blocage. Deux vis convergentes assurent à elles seules un blocage optimal, les deux autres vis opposées pouvant être omises du fait qu'elles ne sont pas nécessaires.

De la même manière la vis 11 (fig. 1) opère une prise efficace sur la partie plane centrale non filetée de la douille 6 qui de cette façon est maintenue rigidement fixée au dé d'articulation 1.

Sur les surfaces planes prévues rugueuses des barres porteuses 4 et 8 sont montées, comme on l'a dit plus haut, les tiges 5, respectivement 9, et ce à l'aide de vis 12 (fig. 5) fixées à la barre correspondante au moyen d'un écrou 13 avec interposition d'un étrier 14 et d'une rondelle 15. Les pièces 14 et 15 sont revêtues d'une garniture déformable 14', respectivement 15' qui fait prise sur la surface rugueuse des parties de guidage des barres porteuses 4 et 8.

La rondelle métallique usuelle 16 qui est directement associée à l'écrou 13 assure un serrage efficace de celui-ci sur l'étrier 14.

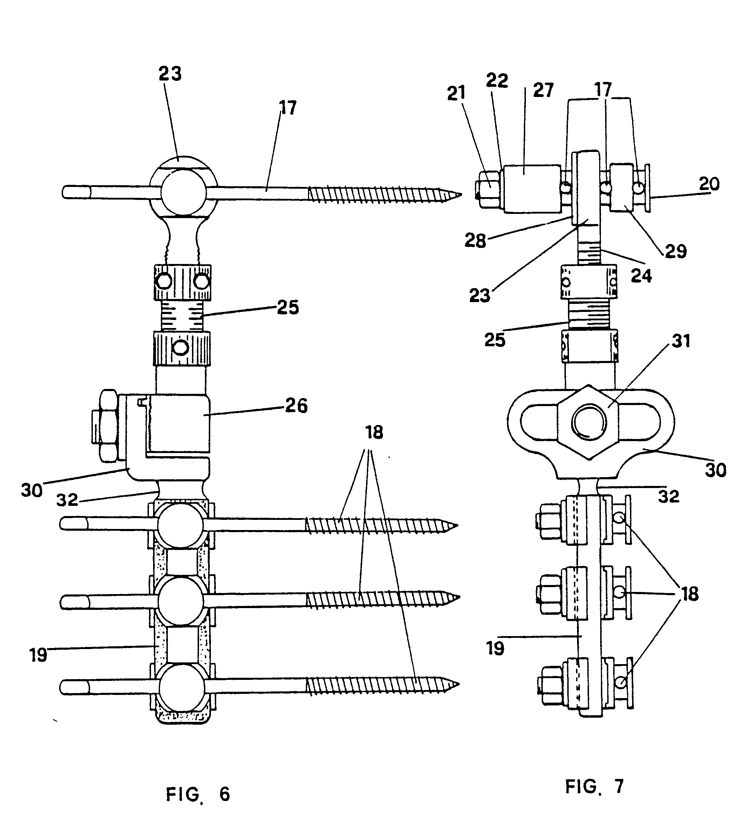

En fig. 6 et 7 on notera que la série formée par les tiges 17 est disposée suivant un plan perpendiculaire à la série formée par les tiges 18, du fait que suivant cette forme de réalisation, le dispositif suivant l'invention est destiné à la stabilisation de fractures osseuses épiphysaires.

En pareil cas le dispositif comprend une barre porteuse unique, référencée 19, qui est destinée à supporter les tiges 18 à introduire dans la diaphyse osseuse, tandis que les tiges 17 à insérer dans l'épiphyse sont simplement fixées à un axe 20 percé par des trous muni d'un écrou 21 et d'une rondelle 22 ; cet écrou 21 opère le blocage des tiges 17 par rapport à un œillet 23 prévu sur la partie filetée 24 de la barre porteuse, cette partie filetée étant à son tour réunie, par une douille 25 et une articulation 26, à la partie de guidage 19 de la barre considérée.

De simples cannelures à section en arc-de-cercle sont ménagées sur le dé 27, sur la rondelle 28 et sur le dé opposé 29, ces pièces étant séparées les unes des autres par les tiges 17. Les tiges 18 sont de leur côté fixées à la partie de guidage 19 de la barre porteuse à l'aide de boulons associés à des écrous, à des rondelles et à des étriers à revêtement lisse, analogues à ce qui a été décrit ci-dessus pour la première forme de l'appareillage correspondant aux fig. 1 à 5.

Dans ce cas toutefois, alors que les tiges 17 sont reliées au dé d'articulation 26 par la douille 25 dont la position peut être réglée de façon à permettre l'allongement de la distance entre l'épiphyse et la diaphyse, la partie de guidage 19 se raccorde, au secteur 30 lui-même fixé en place sur le dé 26 à l'aide d'un écrou 31, par l'intermédiaire d'un col 32 dont le profil curviligne est propre à éviter tout risque de rupture dangereuse pour la guérison du patient.

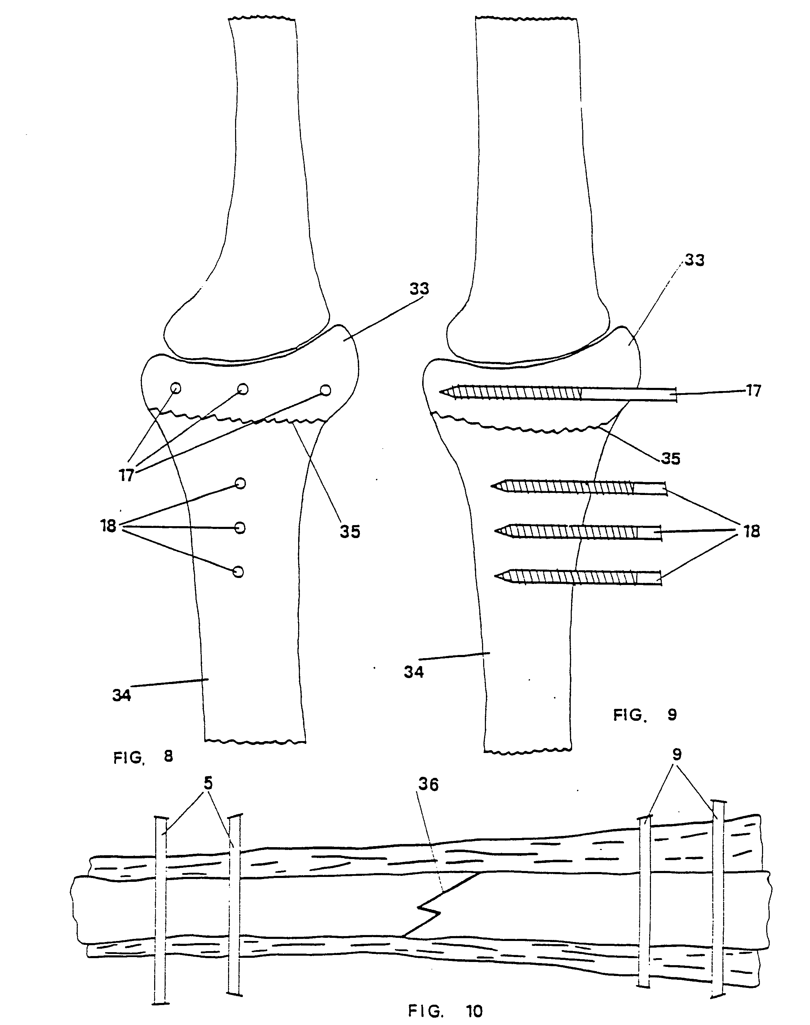

En fig. 9 et 10 on a indiqué les points dans lesquels les tiges 17 doivent être engagées dans l'épiphyse 33. Les tiges 18 pénètrent dans la diaphyse 34 de l'os, la ligne de fracture ayant été schématiquement représentée en 35.

Dans le cas de la fracture de la partie centrale d'un os long, les tiges 5 et 9 sont introduites dans les zones disposées de part et d'autre de la ligne de fracture intermédiaire 36, comme illustré en fig. 10.

On remarquera que l'appareillage ci-dessus décrit peut être avantageusement utilisé pour l'allongement artificiel des membres, et ce moyennant le réglage périodique de la position des douilles filetées 6 ou 25 de façon à augmenter la distance séparant les tronçons osseux en fonction de leur croissance subséquente, conformément à des traitements bien connus en pratique.

Il va de soi que ce réglage est facilité par la présence des petites vis 10″, 10‴ et 11 de fig. 1 qui une fois desserrées autorisent l'allongement du dispositif moyennant une simple rotation d'un tour ou d'un demi-tour de la douille 6, la stabilisation étant ainsi assurée pour la période subséquente de croissance de l'os.

On conçoit par ailleurs que les détails de réalisation du dispositif, tels que le nombre et la position des tiges, les dimensions des différents éléments et la finition superficielle peuvent être quelconques.

## Revendications

1. Appareillage pour la stabilisation des fractures osseuses du genre comprenant deux barres porteuses (4, 8, 19) articulées, des tiges transosseuses destinées à être insérées dans les fragments osseux élémentaires, et au moins un élément (12) de fixation des tiges sur les barres (4, 8, 19), caractérisé en ce qu'il comprend :
    des surfaces rugueuses sur les barres porteuses,
    pour chaque élément (12) de fixation, un mécanisme de blocage de cet élément (12) sur une des barres (4, 8, 19) qui est composé d'un étrier (14) et d'une rondelle (15) situées de part et d'autre de cette barre, dont au moins les surfaces en contact avec les surfaces rugueuses de cette barre sont revêtues d'une garniture déformable, et d'un élément de serrage (13) de l'étrier (14) et de la rondelle (15) sur l'élément de fixation (12).

2. Appareillage suivant la revendication 1, caractérisé en ce que chacune des barres porteuses présente une partie de guidage et une partie continue de blocage et que le raccordement de la partie de guidage et de la partie contiguë de blocage présente un profil curviligne.

3. Appareillage suivant l'une quelconque des revendications 1 et 2, caractérisé en ce qu'une des barres porteuses présente une partie filetée (7) de section ovoïdale qui est seulement filetée sur les deux surfaces curvilignes opposées, afin de permettre de fixer ladite partie à une douille (6) à l'aide d'au moins deux vis (10″, 10‴) dont les axes convergent en direction de la partie filetée (7), en vue de permettre le réglage exact de l'orientation angulaire de cette barre porteuse par rapport à l'autre en fonction des caractéristiques particulières de la fracture à traiter.

4. Appareillage suivant la revendication 3, caractérisé en ce qu'une seulement des barres porteuses est reliée à un dé central d'articulation (1) à l'aide de la douille filetée (6), l'autre barre (4) étant directement solidaire d'un étrier entaillé en forme de secteur relié par une vis audit dé (1) sur lequel la première barre (8) vient se fixer avec interposition de ladite douille (6), de façon à rigidifier l'ensemble.

## Claims

1. Apparatus for the stabilization of bone fractures, of the type comprising two articulated carrier bars (4, 8, 19), trans-osseous rods intended for insertion into the elementary bone fragments, and at least one means (12) for attaching the rods to the bars (4, 8, 19), characterized by comprising the following :
    roughened surfaces on the carrier bars,
    for each attachment means (12), a device for locking said means (12) on one of the bars (4, 8, 19), said device being composed of a bracket (14) and a washer (15) which are situated on either side of said bar, of which at least the surfaces in contact with the roughened surfaces of said bar are covered by a deformable fitting, and further composed of a means (13) for clamping the bracket (14) and the washer (15) on the attachment means (12).

2. Apparatus according to claim 1, characterized in that each of the carrier bars has a guide portion and a contiguous locking portion and that the connection of the guide portion and contiguous locking portion presents a curvilinear profile.

3. Apparatus according to either one of claims 1 and 2, characterized in that one of the carrier bars has a threaded portion (7) of ovate section which is only threaded on the two opposed curvilinear surfaces, in order to enable said portion to be attached to a sleeve (6) by means of at least two screws (10″, 10‴) of which the axes converge in the direction of the threaded portion (7), with a view to permitting exact adjustment of the angular orientation of said carrier bar with respect to the other in response to the particular characteristics of the fracture to be treated.

4. Apparatus according to claim 3, characterized in that one only of the carrier bars is joined to a central articulation die (1) by means of the threaded sleeve (6), the other bar (4) being directly fast with a slotted, sector-shaped bracket joined by a screw to said die (1) upon which the first bar (8) is attached by interposing said sleeve (6), thereby rendering the assembly rigid.

**Patentansprüche**

1. Gerät zum Fixieren von Knochenbrüchen von der Art, daß es zwei anlenkbare Tragstangen (4, 8, 19), Knochen durchquerende Stäbe, die zum Einlassen in die elementaren Knochenfragmente bestimmt sind, und wenigstens ein Element (12) zum Befestigen der Stäbe an den Tragstangen (4, 8, 19) aufweist, dadurch gekennzeichnet, daß es enthält :

rauhe Oberflächen an den Tragstangen,

für jedes Befestigungselement (12) einen Sperrmechanismus, der dieses Element (12) auf einer der Stangen (4, 8, 19) festhält, und der aus einem Bügel (14) und aus einer Unterlegscheibe (15), die auf beiden Seiten dieser Stange gelegen sind, und deren mindestens mit den rauhen Oberflächen dieser Stange in Berührung stehende Außenflächen mit einem deformierbaren Belag versehen sind, und aus einem Klemmelement (13) des Bügels (14) und der Unterlegscheibe (15) auf dem Befestigungselement (12) zusammengesetzt ist.

2. Gerät gemäß dem Anspruch 1, dadurch gekennzeichnet, daß jede der Tragstangen einen Führungsteil und einen angrenzenden Sperrteil darstellt, und daß die Verbindung des Führungsteiles und des angrenzenden Sperrteiles ein gekrümmtes Profil darbietet.

3. Gerät gemäß einem der Ansprüche 1 und 2, dadurch gekennzeichnet, daß eine der Tragstangen einen mit Gewinde (7) versehenen Teil von fast eiförmigem Schnitt darbietet, der allein an zwei gekrümmten, entgegengesetzten Oberflächen die Gewinde trägt, um den besagten Teil an einer Hülse (6) mit Hilfe mindestens zweier Schrauben (10″, 10‴) befestigen zu können, deren Achsen in Richtung des mit Gewinde (7) versehenen Teiles im Hinblick darauf konvergieren, um die Winkellage dieser Tragstange mit Rücksicht auf den Zusammenhang mit speziellen Eigenschaften des zu behandelnden Bruches exakt einstellen zu können.

4. Gerät gemäß dem Anspruch 3, dadurch gekennzeichnet, daß die eine der Tragstangen allein mit Hilfe der mit einem Gewinde versehenen Hülse (6) mit einem zentralen Gelenk (1) verbunden ist, während die andere Stange (4) direkt für einen Bügel verantwortlich ist, der in Sektorform eingeschnitten und durch eine Schraube mit dem besagten Würfel (1) verbunden ist, an dem die erste Stange (8) unter Zwischenschaltung der besagten Hülse (6) zur Befestigung gelangt, um den Aufbau zu versteifen.

FIG. 1

EP 0 235 138 B1

FIG. 2

FIG. 3

FIG. 4

FIG. 5

FIG. 6

FIG. 7

FIG. 8

FIG. 9

FIG. 10